(19) Europäisches Patentamt
European Patent Office
Office européen des brevets

(11) Veröffentlichungsnummer: **0 373 118 A2**

(12) **EUROPÄISCHE PATENTANMELDUNG**

(21) Anmeldenummer: **89810911.1**

(22) Anmeldetag: **28.11.89**

(51) Int. Cl.[5]: **C07H 13/04, C07H 15/18, C07H 9/04, //C07H5/02**

(30) Priorität: **07.12.88 CH 4529/88**

(43) Veröffentlichungstag der Anmeldung: **13.06.90 Patentblatt 90/24**

(84) Benannte Vertragsstaaten: **CH DE FR GB IT LI NL SE**

(71) Anmelder: **CIBA-GEIGY AG**
**Klybeckstrasse 141**
**CH-4002 Basel(CH)**

(72) Erfinder: **Ernst, Beat, Dr.**
**Obere Egg 6**
**CH-4312 Magden(CH)**

(54) **Verfahren zur Herstellung von geschützten Mono- und Oligozuckerhalogeniden.**

(57) Die Umsetzung von geschützten Mono- und Oligosacchariden oder geschützten Mono- und Oligosaccharid-Derivaten, die eine anomere Hydroxylgruppe enthalten, mit sekundären α-Halogenenaminen liefert in hohen Ausbeuten geschützte Glykosylhalogenide, die wertvolle Zwischenprodukte zur Einführung von Zuckergruppen bei der Synthese von Oligosacchariden, Glykolipiden oder Glykopeptiden darstellen.

EP 0 373 118 A2

# Verfahren zur Herstellung von geschützten Mono- und Oligozuckerhalogeniden

Die Erfindung betrifft ein Verfahren zur Herstellung von geschützten Mono- und Oligozuckerhalogeniden oder halogenierten Mono- oder Oligozukkerderivaten mit anomeren Halogenatomen (Glykosylhalogenide), bei dem man einen eine anomere Hydroxylgruppe enthaltenden geschützten Zucker bzw. ein entsprechendes Zuckerderivat mit einem sekundären α-Halogenenamin umsetzt.

Geschützte Glykosylhalogenide sind wichtige Zwischenprodukte zur Einführung von Zuckergruppen. Für die Herstellung der Glykosylhalogenide sind verschiedene Verfahren unter Verwendung unterschiedlicher Halogenierungsmittel bekannt.

H. Kunz et al. beschreiben in Helv. Chim. Acta, Vol. 68, S. 283-287 (1985) die Herstellung von Glykosylfluoriden unter Verwendung von HF oder Triphenylphosphin/Diethylazodicarboxylat /Triethyloxoniumtetrafluoroborat. W. Rosenbrook et al. schlagen in Tetrahedron Letters, Vol. 26, S. 3-4 (1985) nasses Diethylaminoschwefeltrifluorid als Fluorierungsmittel zur Herstellung von Glykosylfluoriden vor.

S. Hanessian et al. beschreiben in Carbohydr. Res., Vol. 24, S. 45-56 (1972) die Halogenierung der anomeren Hydroxylgruppe von Zuckern mit Triphenylphosphin/N-Chlor-, N-Brom- oder N-Iodsuccinimid.

R. Schmidt et al. beschreiben in Tetrahedron Letters, Vol. 21, S. 1421-1423 (1980) die Chlorierung der anomeren Hydroxylgruppe eines Zuckersäureesters mit Thionylchlorid, und die Bromierung mit $PBr_3$.

F.J. Kronzer et al. beschreiben in Carbohyd. Res., Vol. 34, S. 71-78 (1974) die in situ Herstellung von anomeren Glykosyliodiden durch die Umsetzung von Glykosylchloriden mit NaI.

Gegenstand der Erfindung ist ein Verfahren zur Herstellung von geschützten Zuckerhalogeniden oder halogenierten Zuckerderivaten mit einem anomeren Halogenatom durch Halogenierung der anomeren Hydroxylgruppe, das dadurch gekennzeichnet ist, dass man 1 Aequivalent eines geschützten Zuckers oder eines geschützten Zuckerderivats, die eine anomere Hydroxylgruppe enthalten, in einem inerten Lösungsmittel mit mindestens einem Aequivalent eines sekundären α-Halogenenamins umsetzt.

Das Verfahren wird bevorzugt bei einer Temperatur von -20 bis 80 °C, besonders 0 bis 60 °C durchgeführt.

Vorzugsweise verwendet man als Lösungsmittel einen halogenierten aliphatischen Kohlenwasserstoff, einen aromatischen Kohlenwasserstoff oder einen aliphatischen Ether. Beispiele für inerte Lösungsmittel sind $CH_2Cl_2$, $CHCl_3$, $CCl_4$, 1,2-Dichlorethan, 1,1,2,2-Tetrachlorethan, Benzol, Toluol, Xylol, Diethylether, Dibutylether, Ethylenglykoldimethylether, Tetrahydrofuran und Dioxan.

Das α-Halogenenamin wird vorzugsweise in einer Menge von 1 bis 3, besonders 1,5 bis 2,5 Aequivalenten pro Aequivalent geschütztem Zucker verwendet.

Das Verfahren wird vorteilhaft unter Schutzgasatmosphäre durchgeführt, z.B. unter einer $N_2$- oder Edelgasatmosphäre.

Eine bevorzugte Ausführungsform des Verfahrens ist dadurch gekennzeichnet, dass das α-Halogenenamin der Formel I

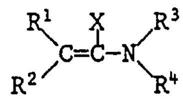

(I)

entspricht, worin $R^1$ und $R^2$ unabhängig voneinander lineares oder verzweigtes $C_1$-$C_{12}$-Alkyl, $C_4$-$C_7$-Cycloalkyl, $C_1$-$C_{12}$-Alkyl-$C_4$-$C_7$-cycloalkyl, $C_4$-$C_7$-Cycloalkylmethyl, $C_1$-$C_{12}$-Alkyl-$C_4$-$C_7$-cycloalkylmethyl, Phenyl, $C_1$-$C_{12}$-Alkylphenyl, Benzyl oder $C_1$-$C_{12}$-Alkylbenzyl bedeuten, oder $R^1$ und $R^2$ zusammen für Tetra-, Penta- oder Hexamethylen stehen; $R^3$ und $R^4$ unabhängig voneinander lineares oder verzweigtes $C_1$-$C_{12}$-Alkyl, $C_4$-$C_7$-Cycloalkyl, $C_1$-$C_{12}$-Alkyl-$C_4$-$C_7$-cycloalkyl, $C_4$-$C_7$-Cycloalkylmethyl, $C_1$-$C_{12}$-Alkyl-$C_4$-$C_7$-cycloalkylmethyl, Phenyl oder Benzyl darstellen, oder $R^3$ und $R^4$ zusammen Tetramethylen, Pentamethylen oder 3-Oxa-1,5-pentylen sind; und X für Halogen steht.

Bei $R^1$, $R^2$, $R^3$ und $R^4$ als Alkyl kann es sich zum Beispiel um Methyl, Ethyl und die Isomeren von Propyl, Butyl, Pentyl, Hexyl, Heptyl, Octyl, Nonyl, Decyl, Undecyl und Dodecyl handeln.

Bei $R^1$, $R^2$, $R^3$ und $R^4$ als Cycloalkyl kann es sich z.B. um Cyclobutyl, Cyclopentyl, Cyclohexyl und Cycloheptyl handeln. Bevorzugt sind Cyclopentyl und Cyclohexyl.

$R^1$, $R^2$, $R^3$ und $R^4$ sind als Alkylcycloalkyl bevorzugt $C_1$-$C_6$-Alkylcyclopentyl und besonders $C_1$-$C_6$-Alkylcyclohexyl. Einige Beispiele sind Methylcyclobutyl, Methylcyclopentyl, Ethylcyclopentyl, Methylcyclohexyl, Ethylcyclohexyl, n-, i- oder t-Butylcyclohexyl, Dimethylcyclohexyl, Methyl-ethyl-cyclohexyl.

$R^1$, $R^2$, $R^3$ und $R^4$ können als Cycloalkylmethyl z.B. Cyclopentylmethyl und Cyclohexylmethyl sein.

Bei $R^1$, $R^2$, $R^3$ und $R^4$ als Alkylcycloalkylmethyl kann es sich z.B. um $C_1$-$C_6$-Alkylcyclopentylmethyl oder $C_1$-$C_6$-Alkylcyclohexylmethyl handeln. Beispiele sind (Methylcyclopentyl)methyl,

(Methylcyclohexyl)methyl, (Butylcyclohexyl)methyl und (Dimethylcyclohexyl)methyl.

Bei $R^1$ und $R^2$ als Alkylphenyl handelt es sich bevorzugt um $C_1$-$C_6$-Alkylphenyl, und als Alkylbenzyl handelt es sich bevorzugt um $C_1$-$C_6$-Alkylbenzyl. Beispiele sind Methylphenyl, Dimethylphenyl, Ethylphenyl, n- oder i-Propylphenyl, n-, i- oder t-Butylphenyl, Methylbenzyl, Dimethylbenzyl, Ethylbenzyl, n- oder i-Propylbenzyl, n-, i-oder t-Butylbenzyl.

X bedeutet als Halogen -F, -Cl, -Br oder -I.

$R^1$ und $R^2$ sowie $R^3$ und $R^4$ stellen bevorzugt unabhängig voneinander $C_1$-$C_6$-Alkyl dar. Besonders bevorzugt stehen $R^1$ und $R^2$ je für Methyl. $R^3$ und $R^4$ bedeuten besonders $C_1$-$C_4$-Alkyl. Insbesondere sind $R^3$ und $R^4$ gleich und bedeuten Methyl, Ethyl, n- oder i-Propyl.

Die Herstellung von sekundären α-Chlorenaminen ist in Organic Synthesis 59, S. 26-34 (1979) beschrieben. Daraus können α-Fluor-, α-Brom- und α-Iodenamine gemäss dem von L. Ghosez et al. in J. Chem. Soc. Chem. Comm., Seite 1180 (1979) beschriebenen Verfahren hergestellt werden.

Die im erfindungsgemässen Verfahren verwendeten geschützten Zucker sind bekannt, nach bekannten Verfahren herstellbar und viele der Zucker sind kommerziell erhältlich. Es kann sich z.B. um geschützte Mono- und Oligosaccharide handeln, z.B. Mono-, Di-, Tri-, Tetra- und Pentasaccharide.

In einer bevorzugten Ausführungsform handelt es sich bei dem geschützten Mono- und Oligosacchariden um eine Aldose oder Ketose mit einer anomeren Hydroxylgruppe.

In einer besonders bevorzugten Ausführungsform handelt es sich bei dem geschützten Monosaccharid um Aldopyranosen, Aldofuranosen, Ketopyranosen oder Ketofuranosen mit einer anomeren Hydroxylgruppe.

Insbesondere ist die Aldopyranose D-Ribose, D-Arabinose, D-Xylose, D-Lyxose, D-Allose, D-Altrose, D-Glucose, D-Mannose, D-Gulose, D-Idose, D-Galaktose oder D-Talose; die Aldofuranose D-Erythrose, D-Threose, D-Ribose, D-Arabinose, D-Xylose, D-Lyxose, D-Allose, D-Altrose, D-Glucose, D-Mannose, D-Gulose, D-Idose, D-Galaktose oder D-Talose; die Ketopyranose D-Psicose, D-Fructose, D-Sorbose oder D-Tagatose; und die Ketofuranose D-Psicose, D-Fructose, D-Sorbose oder D-Tagatose, deren Hydroxylgruppen ausser der anomeren Hydroxylgruppe geschützt sind.

Insbesondere ist das Disaccharid Trehalose, Sophorose, Kojibiose Laminaribiose, Maltose, Cellobiose, Isomaltose, Gentobiose, Saccharose, Raffinose und Lactose, deren Hydroxygruppen ausser der anomeren Hydroxygruppe geschützt sind.

Geschützte Mono- und Oligosaccharid-Derivate mit einer anomeren Hydroxylgruppe sind bekannt, nach bekannten Verfahren herstellbar und teilweise käuflich. Es kann sich z.B. um geschützte Desoxyzucker, Aminozucker, Thiozucker, Zuckersäuren oder Ester von Zuckersäuren handeln, die eine anomere Hydroxylgruppe enthalten, wie z.B. 2-Deoxyzucker, 2-Thiozucker, 2-Aminozucker, Gluconsäuren und deren Ester, bevorzugt deren $C_1$-$C_4$-Alkylester.

Geschützt bedeutet, dass die Hydroxylgruppen der Mono- und Oligosaccharide bzw Mono- und Oligosaccharid-Derivate ausser der anomeren Hydroxylgruppe mit einer abspaltbaren Schutzgruppe derivatisiert sind. Solche Schutzgruppen und Verfahren zur Derivatisierung sind in der Zuckerchemie allgemein bekannt. Beispiele für solche Schutzgruppen sind: Lineares oder verzweigtes $C_1$-$C_8$-, besonders $C_1$-$C_4$-Alkyl, z.B. Methyl, Ethyl, n- und i- Propyl, n-, i- und t-Butyl; $C_7$-$C_{12}$-Aralkyl, z.B. Benzyl, Trialkylsilyl mit 3 bis 20, besonders 3 bis 10 C-Atomen, z.B. Trimethylsilyl, Triethylsilyl, Tri-n-propylsilyl, i-Propyl-dimethylsilyl, t-Butyl-dimethylsilyl, n-Octyl-dimethylsilyl, (1,1,2,2-Tetramethylethyl)-dimethylsilyl;

substituierte Methylidengruppen, die durch Acetal- bzw. Ketalbildung von benachbarten OH-Gruppen der Zucker bzw. Zuckerderivate mit Aldehyden und Ketonen erhältlich sind, die vorzugsweise 2 bis 12, bzw. 3 bis 12 C-Atome enthalten, z.B. $C_1$-$C_{12}$-, bevorzugt $C_1$-$C_6$-, besonders $C_1$-$C_4$-Alkyliden oder Benzyliden (Ethyliden, 1,1- oder 2,2-Propyliden, 1,1- oder 2,2-Butyliden);

$C_2$-$C_{12}$-, besonders $C_2$-$C_8$-Acyl, wie z.B. Acetyl, Propanoyl, Butanoyl, Pentanoyl, Hexanoyl und Benzoyl;

$R^5$-$SO_2$-, worin $R^5$ $C_1$-$C_{12}$-Alkyl, besonders $C_1$-$C_6$-Alkyl, $C_5$- oder $C_6$-Cycloalkyl, Phenyl, Benzyl oder $C_1$-$C_{12}$-Alkylphenyl, besonders $C_1$-$C_4$-Alkylphenyl, oder $C_1$-$C_{12}$-Alkylbenzyl, besonders $C_1$-$C_4$-Alkylbenzyl bedeutet, z.B. Methylsulfonyl, Ethylsulfonyl, Propylsulfonyl, Butylsulfonyl, Phenylsulfonyl, Benzylsulfonyl und p-Methylphenylsulfonyl.

Das erfindungsgemässe Verfahren kann z.B. so durchgeführt werden, dass man 1 Aequivalent eines geschützten Mono- oder Oligosaccharids bzw. Mono-oder Oligosaccharid-Derivats in einem Lösungsmittel löst und vorzugsweise unter Schutzgasatmosphäre (Stickstoff, Argon) mit mindestens 1 Aequiva lent sekundärem α-Halogenenamin versetzt, wobei vorteilhaft eine Temperatur von -20 bis +20° C, besonders um 0° C, eingehalten wird. Dann lässt man bis zum vollständigen Umsatz weiterreagieren, vorteilhaft bei erhöhter Temperatur von z.B. bis 80° C.

Zur Isolierung der Glykosylhalogenide kann z.B. zunächst das Reaktionsgemisch durch Destillation gegebenenfalls im Vakuum eingeengt werden. Der Rückstand kann entweder direkt zu Glykolisierungsreaktionen eingesetzt oder mit Hilfe üblicher Methoden (Destillation, Kristallisation oder

chromatographische Methoden) weiter gereinigt werden.

Mit dem erfindungsgemässen Verfahren können überraschend alle Glykosylhalogenide mit nur einem Halogenierungsmittel hergestellt werden, wobei gleichzeitig hohe Ausbeuten und Selektivitäten erzielt werden. Die Reaktion verläuft unter vollständig neutralen Bedingungen, so dass auch Zukker mit säurelabilen und baselabilen Schutzgruppen verwendet werden können, oder auch Zuckerderivate, die solche labile Gruppen enthalten, z.B. Silylethergruppen. Mit dem erfindungsgemässen Verfahren kann ferner die autokatalytische Zersetzung der Brom- und Iodglykoside vermieden werden, die bei anderen Herstellverfahren beobachtet wird.

Die Glykosylhalogenide eignen sich zur Einführung von Zuckergruppen bei der Synthese von Oligosacchariden, Glykolipiden oder Glykopeptiden, siehe z.B. H. Paulsen, Chem. Soc. Rev., 13, S. 15-45 (1984) und R.R. Schmidt, Angew. Chem. 98, 213-236 (1986).

Die nachfolgenden Beispiele erläutern die Erfindung näher.

Beispiel 1: 513 mg (1,47 mMol) 2,3,4,6-Tetra-O-acetyl-mannopyranose in 5 ml trockenem Chloroform werden bei 0 °C unter Argon mit 229 µl (1,62 mMol) 1-Chlor-N,N,2-trimethylpropenylamin versetzt. Anschliessend wird 20 h. bei Raumtemperatur gerührt. Das Reaktionsgemisch wird eingeengt und an Kieselgel 60 mit Petroleumbenzin 40-60 °/Essigsäureethylester (2:1) chromatographiert. Dabei werden 406 mg (75 %) 2,3,4,6-Tetra-O-acetyl-α-D-mannopyranosylchlorid erhalten.

$^1$H-NMR (300 MHz, $CDCl_3$): 5,99 (d, J = 1,7, H-C-(1)).

Beispiel 2: Analog Beispiel 1 werden ausgehend von 521 mg (1,49 mMol) 2,3,4,6-Tetra-O-acetyl-mannopyranose mit 234 µl (1,64 mMol) 1-Brom-N,N,2-trimethylpropenylamin 514 mg (84 %) 2,3,4,6-Tetra-O-acetyl-α-D-mannopyranosylbromid erhalten.

$^1$H-NMR (300 MHz, $CDCl_3$): 6,30 (d, J = 1,6, H-C-(1)).

Beispiel 3: Analog zu Beispiel 1 werden ausgehend von 527 mg (1,51 mMol) 2,3,4,6-Tetra-O-acetyl-mannopyranose mit 272 µl (1,66 mMol) 1-Iod-N,N,2-trimethylpropenylamin 545 mg (79 %) 2,3,4,6-Tetra-O-acetyl-α-D-mannopyranosyliodid erhalten.

$^1$H-NMR (300 MHz, $CDCl_3$): 6,71 (d, J = 1,6, H-C-(1)).

Beispiel 4: Analog zu Beispiel 1 werden ausgehend von 455 mg (1,30 mMol) 2,3,4,6-Tetra-O-acetyl-mannopyranose mit 289 µl (1,43 mMol) 1-Fluor-N,N-diisopropyl-2-methylpropenylamin 450 mg (98 %, α:β = 6:1) 2,3,4,6-Tetra-O-acetyl-α-D-mannopyranosylfluorid erhalten.

$^1$H-NMR (300 MHz, $CDCl_3$): 5,58 (dd, $J_{1,F}$ = 48,5, $J_{1,2}$ = 1,9, β-H-C(1)); 5,55 (dd, $J_{1,F}$ = 49,5, $J_{1,2}$ = 1,7, α-H-C(1)).

Beispiel 5: Analog zu Beispiel 1 werden ausgehend von 1,00 g (2,87 mMol) 2,3,4,6-Tetra-O-acetyl-glucopyranose mit 0,81 ml (5,74 mMol) 1-Chlor-N,N,2-trimethylpropenylamin 964 mg (91 %) 2,3,4,6-Tetra-O-acetyl-α-D-glucopyranosylchlorid erhalten.

$^1$H-NMR (300 MHz, $CDCl_3$): 6,30 (d, J = 3,9, H-C-(1)).

Beispiel 6: Analog zu Beispiel 1 werden ausgehend von 494 mg (1,41 mMol) 2,3,4,6-Tetra-O-acetyl-glucopyranose mit 201 µl (1,41 mMol) 1-Brom-N,N,2-trimethylpropenylamin 450 mg (77 %) 2,3,4,6-Tetra-O-acetyl-α-D-glucopyranosylbromid erhalten.

$^1$H-NMR (300 MHz, $CDCl_3$): 6,61 (d, J = 4, H-C(1)-).

Beispiel 7: Analog Beispiel 1 werden ausgehend von 506 mg (1,45 mMol) 2,3,4,6-Tetra-O-acetyl-glucopyranose mit 475 µl (2,90 mMol) 1-Iod-N,N,2-trimethylpropenylamin 556 mg (84 %) 2,3,4,6-Tetra-O-acetyl-α-D-glucopyranosyliodid erhalten.

$^1$H-NMR (300 MHz, $CDCl_3$): 6,99 (d, J = 4, H-C(1)-).

Beispiel 8: Analog Beispiel 1 werden ausgehend von 455 mg (1,30 mMol) 2,3,4,6-Tetra-O-acetyl-glucopyranose mit 527 µl (2,61 mMol) 1-Fluor-N,N-diisopropyl-2-methylpropenylamin 386 mg (85 %, α:β = 1:3) 2,3,4,6-Tetra-O-acetyl-α-D-glucopyranosylfluorid erhalten.

$^1$H-NMR (300 MHz, $CDCl_3$): 5,75 (dd, $J_{1,F}$ = 53, $J_{1,2}$ = 3, β-H-C(1)); 5,37 (dd, $J_{1,F}$ = 52, $J_{1,2}$ = 6, α-H-C(1)).

Beispiel 9: Analog Beispiel 1 werden ausgehend von 540 mg (1,0 mMol) 2,3,4,6-Tetra-O-benzyl-D-glucopyranose mit 156 µl (1,1 mMol) 1-Chlor-N,N,2-trimethylpropenylamin 517 mg (92 %) 2,3,4,6-Tetra-O-benzyl-α-D-glucopyranosylchlorid erhalten.

$^1$H-NMR (300 MHz, $CDCl_3$): 6,06 (d, J = 3,7, H-C-(1)).

Beispiel 10: Analog Beispiel 1 werden ausgehend von 203 mg (0,37 mMol) 2,3,4,6-Tetra-O-benzyl-D-glucopyranose mit 80 µl (0,56 mMol) 1-Brom-N,N,2-trimethylpropenylamin 224 mg (98 %) 2,3,4,6-Tetra-O-benzyl-α-D-glucopyranosylbromid erhalten.

$^1$H-NMR (300 MHz, $CDCl_3$): 6,43 (d, J = 3,7, H-C-(1)).

Beispiel 11: Analog Beispiel 1 werden ausgehend von 500 mg (0,925 mMol) 2,3,4,6-Tetra-O-benzyl-D-glucopyranose mit 373 µl (1,85 mMol) 1-Fluor-N,N-diisopropyl-2-methylpropenylamin 498 mg (99 %, α:β = 28:72) 2,3,4,6-Tetra-O-benzyl-α-D-glucopyranosylfluorid erhalten.

$^1$H-NMR (300 MHz, $CDCl_3$): 5,55 (dd, $J_{1,F}$ = 53, $J_{1,2}$ = 2,8, β-H-C(1)); 5,25 (dd, $J_{1,F}$ = 52,7, $J_{1,2}$ = 6,5, α-H-C(1)).

Beispiel 12: Analog Beispiel 1 werden ausgehend von 381 mg (0,70 mMol) 2,3,4,6-Tetra-O-benzyl-D-glucopyranose mit 346 µl (2,11 mMol) 1-Iod-N,N,2-trimethylpropenylamin nach Einengen des Reaktionsgemisches quantitativ 2,3,4,6-Tetra-O-benzyl-α-D-glucopyranosyliodid erhalten.
$^1$H-NMR (300 MHz, $CDCl_3$): 6,85 (d, J = 4, H-C(1)-).

Beispiel 13: Analog Beispiel 1 werden ausgehend von 198 mg (0,76 mMol) 2,3:5,6-Bis-O-(1-methylethyliden)-α-D-mannofuranose mit 306 µl (1,52 mMol) 1-Fluor-N,N-diisopropyl-2-methylpropenylamin 40 mg (20 %) 2,3:5,6-Bis-O-(1-methylethyliden)-α-D-mannofuranosylfluorid und 114 mg (57 %) 2,3:5,6-Bis-O-(methylethyliden)-β-D-mannofuranosylfluorid erhalten.
$^1$H-NMR (300 MHz, $CDCl_3$): 5,69 (d, $J_{1,F}$ = 59,3, β-H-C(1)); 5,51 (dd, $J_{1,F}$ = 66,6, $J_{1,2}$ = 3,7, α-H-C(1)-).

Beispiel 14: Analog Beispiel 1 werden ausgehend von 203 mg (0,77 mMol) 2,3:5,6-Bis-O-(1-methylethyliden)-α-D-mannofuranose mit 122 µl (0,85 mMol) 1-Chlor-N,N,2-trimethylpropenylamin 170 mg (78 %) 2,3:5,6-Bis-O-(1-methylethyliden)-α-D-mannofuranosylchlorid erhalten.
$^1$H-NMR (300 MHz, $CDCl_3$): 6,08 (s, α-H-C(1)).

Beispiel 15: Analog Beispiel 1 wird ausgehend von 199 mg 2,3:5,6-Bis-O-(1-methylethyliden)-α-D-mannofuranose mit 239 µl (1,68 mMol) 1-Brom-N,N,2-trimethylpropenylamin nach Einengen des Reaktionsgemisches 220 mg (90 %) 2,3:5,6-Bis-O-(1-methylethyliden)-α-D-mannofuranosylbromid erhalten.
$^1$H-NMR (300 MHz, $CDCl_3$): 6,39 (s, α-H-C(1)).

Beispiel 16: Analog Beispiel 1 wird ausgehend von 187 mg (0,71 mMol) 2,3:5,6-Bis-O-(1-methylethyliden)-α-D-mannofuranose mit 235 µl (1,43 mMol) 1-Iod-N,N,2-trimethylpropenylamin nach Einengen des Reaktionsgemisches 192 mg (72 %) 2,3:5,6-Bis-O-(1-methylethyliden)-α-D-mannofuranosyliodid erhalten.
$^1$H-NMR (300 MHz, $CDCl_3$): 6,63 (d, J = 1, β-H-C-(1)).

Beispiel 17: Analog Beispiel 1 werden ausgehend von 210 mg (0,62 mMol) 2,3,4-Tri-O-acetyl-D-glucuronsäuremethylester mit 253 µl (1,25 mMol) 1-Fluor-N,N-diisopropyl-2-methylpropenylamin 161 mg (76 %) 2,3,4-Tri-O-acetyl-1-deoxy-1-fluor-D-glucuronsäuremethylester (β:α = 9:1) erhalten.
$^1$H-NMR (300 MHz, $CDCl_3$): 5,82 (dd, $J_{1,F}$ = 53, $J_{1,2}$ = 3, β-H-C(1)); 5,45 (dd, $J_{1,F}$ = 51, $J_{1,2}$ = 5, α-H-C(1)).

Beispiel 18: Analog Beispiel 1 werden ausgehend von 191 mg (0,57 mMol) 2,3,4-Tri-O-acetyl-D-glucuronsäuremethylester mit 89 µl (0,62 mMol) 1-Chlor-N,N,2-trimethylpropenylamin 169 mg (88 %) 2,3,4-Tri-O-acetyl-1-deoxy-1-chlor-D-glucuronsäuremethylester (α:β = 18:82) erhalten.
$^1$H-NMR (300 MHz, $CDCl_3$): 6,34 (d, J = 4, β-H-C-(1)); 5,36 (d, J = 8, α-H-C(1)).

Beispiel 19: Analog Beispiel 1 wird ausgehend von 199 mg (0,59 mMol) 2,3,4-Tri-O-acetyl-D-glucuronsäuremethylester mit 186 µl (1,30 mMol) 1-Brom-N,N,2-trimethylpropenylamin nach Einengen des Reaktionsgemischs quanititativ 2,3,4-Tri-O-acetyl-1-deoxy-1-brom-α-D-glucuronsäuremethylester erhalten.
$^1$H-NMR (300 MHz, $CDCl_3$): 6,64 (d, J = 4, H-C(1)-).

Beispiel 20: Analog Beispiel 1 wird ausgehend von 227 mg (0,67 mMol) 2,3,4-Tri-O-acetyl-D-glucuronsäuremethylester mit 333 µl (2,03 mMol) 1-Iod-N,N,2-trimethylpropenylamin nach Einengen des Reaktionsgemischs quantitativ 2,3,4-Tri-O-acetyl-1-deoxy-1-iod-α-D-glucuronsäuremethylester erhalten.
$^1$H-NMR (300 MHz, $CDCl_3$): 7,02 (d, J = 4,4, H-C-(1)).

Beispiel 21: Analog Beispiel 1 wird ausgehend von 433 mg (0,68 mMol) Heptaacetylcellobiose mit 274 µl (1,36 mMol) 1-Fluor-N,N-diisopropyl-2-methylpropenylamin 388 mg (90 %) Heptaacetylcellobiosylfluorid erhalten.
$^1$H-NMR (300 MHz, $CDCl_3$): 5,36 (dd, $J_{1,F}$ = 52,7, $J_{1,2}$ = 5,6; H-C-C1).

## Ansprüche

1. Verfahren zur Herstellung von geschützten Zuckerhalogeniden oder halogenierten Zuckerderivaten mit einem anomeren Halogenatom durch Halogenierung der anomeren Hydroxylgruppe, dadurch gekennzeichnet, dass man 1 Aequivalent eines geschützten Zuckers oder eines geschützten Zuckerderivats, die eine anomere Hydroxylgruppe enthalten, in einem inerten Lösungsmittel mit mindestens einem Aequivalent eines sekundären α-Halogenenamins umsetzt.

2. Verfahren gemäss Anspruch 1, dadurch gekennzeichnet, dass es bei einer Temperatur von -20 bis 80° C durchgeführt wird.

3. Verfahren gemäss Anspruch 1, dadurch gekennzeichnet, dass man als Lösungsmittel einen halogenierten aliphatischen Kohlenwasserstoff, einen aromatischen Kohlenwasserstoff oder einen aliphatischen Ether verwendet.

4. Verfahren gemäss Anspruch 1, dadurch gekennzeichnet, dass 1 bis 3 Aequivalente des α-Halogenenamins verwendet werden.

5. Verfahren gemäss Anspruch 1, dadurch gekennzeichnet, dass es unter Schutzgasatmosphäre durchgeführt wird.

6. Verfahren gemäss Anspruch 1, dadurch gekennzeichnet, dass das α-Halogenenamin der Formel I

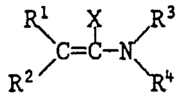

(I)

entspricht, worin $R^1$ und $R^2$ unabhängig voneinander lineares oder verzweigtes $C_1$-$C_{12}$-Alkyl, $C_4$-$C_7$-Cycloalkyl, $C_1$-$C_{12}$-Alkyl-$C_4$-$C_7$-cycloalkyl, $C_4$-$C_7$-Cycloalkylmethyl, $C_1$-$C_{12}$-Alkyl-$C_4$-$C_7$-cycloalkylmethyl, Phenyl, $C_1$-$C_{12}$-Alkylphenyl, Benzyl oder $C_1$-$C_{12}$-Alkylbenzyl bedeuten, oder $R^1$ und $R^2$ zusammen für Tetra-, Penta- oder Hexamethylen stehen; $R^3$ und $R^4$ unabhängig voneinander lineares oder verzweigtes $C_1$-$C_{12}$-Alkyl, $C_4$-$C_7$-Cycloalkyl, $C_1$-$C_{12}$-Alkyl-$C_4$-$C_7$-cycloalkyl, $C_4$-$C_7$-Cycloalkylmethyl, $C_1$-$C_{12}$-Alkyl-$C_4$-$C_7$-cycloalkylmethyl, Phenyl oder Benzyl darstellen, oder $R^3$ und $R^4$ zusammen Tetramethylen, Pentamethylen oder 3-Oxa-1,5-pentylen sind; und X für Halogen steht.

7. Verfahren gemäss Anspruch 6, dadurch gekennzeichnet, dass $R^1$ und $R^2$ unabhängig voneinander $C_1$-$C_6$-Alkyl darstellen.

8. Verfahren gemäss Anspruch 7, dadurch gekennzeichnet, dass $R^1$ und $R^2$ je für Methyl stehen.

9. Verfahren gemäss Anspruch 6, dadurch gekennzeichnet, dass $R^3$ und $R^4$ unabhängig voneinander $C_1$-$C_6$-Alkyl darstellen.

10. Verfahren gemäss Anspruch 9, dadurch gekennzeichnet, dass $R^3$ und $R^4$ je für $C_1$-$C_4$-Alkyl stehen.

11. Verfahren gemäss Anspruch 9, dadurch gekennzeichnet, dass $R^3$ und $R^4$ gleich sind und für Methyl, Ethyl, n- oder i-Propyl stehen.

12. Verfahren gemäss Anspruch 1, dadurch gekennzeichnet, dass es sich bei dem geschützten Zucker oder Zuckerderivat um Mono- oder Oligosaccharide oder Mono- oder Oligosaccharid-Derivate handelt.

13. Verfahren gemäss Anspruch 12, dadurch gekennzeichnet, dass es sich bei dem geschützten Mono- oder Oligosaccharid um eine Aldose oder Ketose mit einer anomeren Hydroxylgruppe handelt.

14. Verfahren gemäss Anspruch 12, dadurch gekennzeichnet, dass es sich bei den geschützten Mono- oder Oligosaccharid-Derivaten um Desoxyzucker, Aminozucker, Thiozucker, Zuckersäuren oder Ester von Zuckersäuren mit einer anomeren Hydroxylgruppe handelt.

15. Verfahren gemäss Anspruch 12, dadurch gekennzeichnet, dass es sich bei dem geschützten Mono- oder Oligosacchariden um Aldopyranosen, Aldofuranosen, Ketopyranosen oder Ketofuranosen mit einer anomeren Hydroxylgruppe handelt.

16. Verfahren gemäss Anspruch 15, dadurch gekennzeichnet, dass die Aldopyranose D-Ribose, D-Arabinose, D-Xylose, D-Lyxose, D-Allose, D-Altrose, D-Glucose, D-Mannose, D-Gulose, D-Idose, D-Galaktose oder D-Talose; die Aldofuranose D-Erythrose, D-Threose, D-Ribose, D-Arabinose, D-Xylose, D-Lyxose, D-Allose, D-Altrose, D-Glucose, D-Mannose, D-Gulose, D-Idose, D-Galaktose oder D-Talose ist; die Ketopyranose D-Psicose, D-Fructose, D-Sorbose oder D-Tagatose ist; und die Ketofuranose D-Psicose, D-Fructose, D-Sorbose oder D-Tagatose ist, deren Hydroxylgruppen ausser der anomeren Hydroxylgruppe geschützt sind.

17. Verfahren gemäss Anspruch 1, dadurch gekennzeichnet, dass die Hydroxylgruppen des Zuckers oder Zuckerderivats ausser der anomeren Hydroxylgruppe mit $C_1$-$C_8$-Alkyl, Trialkylsilyl mit 3 bis 20 C-Atomen, $C_7$-$C_{12}$-Aralkyl, $C_1$-$C_{12}$-Alkyliden, Benzyliden, $C_2$-$C_{12}$-Acyl oder $R^5$-$SO_2$-geschützt sind, wobei $R^5$ $C_1$-$C_{12}$-Alkyl, $C_5$- oder $C_6$-Cycloalkyl, Phenyl, Benzyl, $C_1$-$C_{12}$-Alkylphenyl oder $C_1$-$C_{12}$-Alkylbenzyl bedeutet.